# EUROPEAN PATENT SPECIFICATION

(11) **EP 2 297 357 B1**
(45) Date of publication and mention of the grant of the patent: **31.10.2012**
(21) Application number: 09769355.0
(22) Date of filing: 26.06.2009
(51) Int. Cl.: C12Q 1/68

(54) **PREDICTION OF ANTIVIRAL THERAPY RESPONSE**
VORHERSAGE DES ANSPRECHENS AUF EINE ANTIVIRALE THERAPIE
PRÉDICTION DE LA RÉPONSE À UNE THÉRAPIE ANTIVIRALE

(30) Priority: 27.06.2008 EP 08159274
(43) Date of publication of application: 23.03.2011
(73) Proprietor: Novartis Forschungsstiftung, Zweigniederlassung Friedrich Miescher Institute For Biomedical Research, 4058 Basel (CH); University Hospital Basel, 4031 Basel (CH)
(72) Inventor: FILIPOWICZ, Witold, CH-4125 Riehen (CH); HEIM, Markus, CH-4104 Oberwil (CH); KROL, Jacek, CH-4052 Basel (CH); MARKIEWICZ, Ilona, CH-4052 Basel (CH); FILIPOWICZ, Magdalena, CH-4051 Basel (CH)
(74) Representative: Favre, Nicolas
(86) International application number: PCT/EP2009/058045
(87) International publication number: WO 2009/156507

(56) References cited:
- PEDERSEN I.M. ET AL.,: "Interferon modulation of cellular microRNAs as an antiviral mechanism" NATURE, vol. 449, 18 October 2007 (2007-10-18), pages 919-922, XP002509428 cited in the application
- JOPLING CATHERINE L ET AL: "Modulation of hepatitis C virus RNA abundance by a liver-specific microRNA" SCIENCE, AMERICAN ASSOCIATION FOR THE ADVANCEMENT OF SCIENCE, US, WASHINGTON, DC, vol. 309, no. 5740, 1 September 2005 (2005-09-01), pages 1577-1581, XP009109384 ISSN: 0036-8075
- LIU CHANG-GONG ET AL: "An oligonucleotide microchip for genome-wide microRNA profiling in human and mouse tissues" PROCEEDINGS OF THE NATIONAL ACADEMY OF SCIENCES OF USA, NATIONAL ACADEMY OF SCIENCE, WASHINGTON, DC.; US, vol. 101, no. 26, 29 June 2004 (2004-06-29), pages 9740-9744, XP002364908 ISSN: 0027-8424
- GOTTWEIN E.& BRYAN R.C.: "viral and cellular MicroRNAs as dterminants of viral pathogenesis and immunity" CELL HOST & MICROBE, vol. 3, 12 June 2008 (2008-06-12), pages 375-387, XP002509429
- SARASIN-FILIPOWICZ M. ET AL.,: "decreased levels of microRNA miR-122 in individuals with hapatitis C responding poorly to interferon therapy" NATURE MEDICINE, vol. 15, January 2009 (2009-01), pages 31-33, XP002541650

## Description

The present invention relates method for determining whether or not antiviral therapies will be effective.

Viral infections represent a serious threat to health and are known to be a major cause of morbidity to animals and man. For instance, Hepatitis C virus (HCV) infection is a major cause of chronic liver disease worldwide. An important and striking feature of hepatitis C is its tendency towards chronicity. In over 70% of infected individuals, HCV establishes a persistent infection over decades that may lead to cirrhosis and hepatocellular carcinoma.

An interesting hypothesis in HCV biology proposes that the viral NS3-4A protease not only processes the viral proteins but also cleaves and inactivates components of the intracellular sensory pathways that detect viral infection and induce the transcriptional activation of type I interferons (IFN). One of the targets of NS3-4A is TRIF (TIR domain-containing adapter inducing IFNβ), an essential link between dsDNA detection in endosomes by TLR3 (toll-like receptor 3) and the induction of IFNβ. More recently, retinoic acid inducible gene-I (RIG-I) and MDA5 (helicard) were identified as intracellular sensors of dsRNA. Both sensors signal through Cardif (also known as IPS-1, MAVS, VISA) to induce IFNβ production. Cardif can be cleaved and inactivated by HCV NS3-4A. Two RNA helicases, RIG-I and MDA5, identified as intracellular sensors of dsRNA act through Cardif to induce IFNβ production.

Type I IFNs are not only crucial components of the innate immune system, but are also the most important components of current therapies against CHC. The current standard therapy consists of pegylated IFNα (pegIFNα) injected once weekly subcutaneously and daily intake of the oral antiviral agent ribavirin. This regimen achieves an overall sustained virological response (SVR) in about 55% of the patients, with significant differences between genotypes. An SVR is defined as the loss of detectable HCV RNA during treatment and its continued absence for at least 6 months after stopping therapy. Several studies of long-term follow-up on patients who achieve an SVR demonstrate that this response is durable in over 95% of patients. The probability of a SVR strongly depends on the early response to treatment. Patients who do not show an early virological response (EVR), defined as a decline of the viral load by at least 2 log₁₀ after 12 weeks of therapy, are highly unlikely to develop an SVR, and treatment can be stopped in these patients. On the other hand, patients with an EVR have a good chance to be cured, with 65% of them achieving a SVR. The prognosis is even better for patients who have a rapid virological response (RVR), defined as serum HCV RNA undetectable after 4 weeks of treatment. Over 85% of them will achieve a SVR. Unfortunately, less than 20% of patients with genotype 1 and about 60% of patients with genotypes 2 or 3 show a RVR. The host factors that are important for an early response to therapy are currently not known.

Type I IFNs achieve their potent antiviral effects through the regulation of hundreds of genes (ISG, interferon stimulated genes). The transcriptional activation of ISGs induces proteins that are usually not synthesized in resting cells and that establish a non-virus-specific antiviral state within the cell. Interferons induce their synthesis by activating the Jak-STAT pathway, a paradigm of cell signaling used by many cytokines and growth factors. All type I IFNs bind to the same cell surface receptor (IFNAR) and activate the receptor-associated Janus kinase family members Jak1 and Tyk2. The kinases then phosphorylate and activate signal transducer and activator of transcription 1 (STAT1) ) and STAT2. The activated STATs translocate into the nucleus where they bind specific DNA elements in the promoters of ISGs. Many of the ISGs have antiviral activity but others are involved in lipid metabolism, apoptosis, protein degradation and inflammatory cell responses. As is the case with many viruses, HCV interferes with the IFN system, probably at multiple levels. IFN induced Jak-STAT signaling is inhibited in cells and transgenic mice that express HCV proteins, and in liver biopsies of patients with CHC. *In vitro,* HCV proteins NS5A and E2 bind and inactivate protein kinase R (PKR), an important non-specific antiviral protein. However, the molecular mechanisms that are important for the response to therapeutically applied IFN in patients with CHC are currently unknown. (Sarasin-Filipowicz et al., 2008, PNAS, 105(19):7034-7039).

The capacity of HCV to interfere with the IFN pathway at many different levels is a likely mechanism underlying HCV success to establish a chronic infection (Gale, M., Jr. & Foy, E.M., Nature 436, 939-945 (2005). However, quite paradoxically, in chimpanzees acutely or chronically infected with HCV hundreds of ISGs are induced in the liver (7 Bigger, C.B., et al. J Virol 78, 13779-13792 (2004)). Nevertheless, despite the activation of the endogenous IFN system, the virus is not cleared from chronically infected animals. The results obtained with chimpanzees are difficult to extrapolate to humans since there are some important differences in the pathobiology of HCV infection between these species. Whereas most chimpanzees acutely infected with HCV clear the virus spontaneously, infections in men mostly become chronic. On the other hand, chronically infected chimpanzees can rarely be cured with IFN, whereas more than half of the patients with CHC are successfully treated.

Induction of ISGs was also found in pre-treatment liver biopsies of many patients with CHC, again demonstrating that HCV infection can lead to activation of the endogenous IFN system (Chen, L., et al. Gastroenterology 128, 1437-1444 (2005)). Notably, patients with pre-elevated expression of ISGs tended to respond poorly to therapy when compared to patients having low initial expression (Chen, L., et al. Gastroenterology 128, 1437-1444 (2005)). The cause of this differential response to therapy is not understood.

The present invention is based upon studies in which the inventors investigated IFN induced signaling and ISG induction in paired samples of liver biopsies and peripheral blood mononuclear cells (PBMCs) of patients with chronic hepatitis before and during therapy with pegIFNα. They further correlated biochemical and molecular data with the response to treatment. Their work is set out in more detail in the accompanying Example.

In a previous study, the inventors established that some subjects with a viral infection of the liver are in a state of "pre-activation", such that the IFN signalling pathway is in a state of stimulation with activated ISGs (Sarasin-Filipowicz et al., 2008, PNAS, 105(19):7034-7039). The inventors have found that such individuals, when subsequently treated with IFN and an antiviral agent, had a poor, or no, response to the antiviral treatment. In contrast, another group of infected subjects appeared to have no prior stimulation of IFN receptors (and stimulation of ISGs) and this group responded well to the antiviral therapy (i.e. they had a rapid virological response (RVR)). Moreover it is possible to determine whether a subject would be a poor responder to treatment or have a RVR according to the expression level of a number of specific genes, some of which are ISG genes. In other words, the inventors identified a set of genes that are prognostic genetic markers, the expression levels of which predict whether a subject will respond to antiviral treatment.

In a further study using paired samples of liver biopsies and peripheral blood mononuclear cells (PBMCs) of patients with chronic hepatitis before and during therapy with pegIFNα, the present inventors investigated the microRNAs (miR) of the patients.

MicroRNAs (miRNAs) are a class of non-coding RNA gene whose final product is a ~22 nt functional RNA molecule. They are processed from endogenously encoded imperfect hairpin precursors as single-stranded RNAs. They appear to function via translational repression through base-pairing to the 3'-untranslated region (UTR) of target mRNAs (Griffith-Jones et al., 2006, Nucleic Acids Research, Vol. 34, D140-D144).

MicroRNA (miRNA) biogenesis is a complex, multi step process. Primary miRNA transcripts are transcribed by RNA polymerase II an can range in size from hundreds to thousands of nucleotides in length (pri-miRNA). MiRNAs can be traced back to two genomic sources. Some miRNAs are located within intronic regions of protein-coding genes. Others are located within the introns or exons of non-coding RNAs. Interestingly, pri-miRNAs can encode for a single miRNA but can also contain clusters of several miRNAs. The pri-miRNA is subsequently processed into a ~70nt hairpin (pre-miRNA) by the nuclear ribonuclease III (RNase III) endonuclease, Drosha. The pre-miRNA is than exported from the nucleus into the cytoplasm by Exporin5/RanGTP. In the cytoplasm, a second RNase III, Dicer, together with its dsRBD protein partner, cuts the pre-miRNA in the stem region of the hairpin thereby liberating an ~21 nucleotide RNA-duplex. From the miRNA duplex, one strand enters the protein complex that repress target gene expression, the RNA-induced silencing complex (RISC), whereas the other strand is degraded. The choice of strand relies on the local thermodynamic stability of the miRNA duplex. The strand whose 5' end is less stably paired is loaded into the RISC complex. The miRNAs loaded into the RISC complex appear to function via translational repression through base-pairing to the 3'-untranslated region (UTR) of target mRNAs Du, T. and Zamore, P.D. et al. micro-Primer: the biogenesis and function of microRNA. Development (2005) 132, 4645-4652. Currently 462 human miRNA sequences are deposited in miRBase (http://microrna.sanger.ac.uk) and it is suggested that this list will reach the 800 mark. The large numbers of miRNAs identified so far suggests that they might play complex roles in the regulation and fine tuning of biological processes. Indeed, several miRNAs have been implicated in cell proliferation control (mir-125b and let-7), hematopoietic B-cell lineage fate (mir-181), B-cell survival (mir-15a and mir-16-1), brain patterning (mir-430), pancreatic cell insulin secretion (mir-357), adipocyte development (mir-375) and muscle proliferation and differentiation (miR-1 and miR-133). Many miRNAs are located in genomic regions involved in cancer. For example, the cluster containing mir-16-1 and mir-15 is deleted and downregulated in the majority of B-cell chronic lymphocytic leukemias (B-CLL; Calin, G.A., et al. MicroRNA-cancer connection: The beginning of a new tale. Cancer Res.(2006) 66, 7390-7394).

miRNAs seem to be involved in gene regulation. Some miRNAs, including lin-4 and let-7, inhibit protein synthesis by binding to partially complementary 3' untranslated regions (3' UTRs) of target mRNAs. Others, including the Scarecrow miRNA found in plants, function like siRNA and bind to perfectly complementary mRNA sequences to destroy the target transcript (Grishok et al, 2001).

Research on microRNAs is increasing as scientists are beginning to appreciate the broad role that these molecules play in the regulation of eukaryotic gene expression. The two best understood miRNAs, lin-4 and let-7, regulate developmental timing in C. elegans by regulating the translation of a family of key mRNAs (reviewed in Pasquinelli, 2002). Several hundred miRNAs have been identified in C. elegans, Drosophila, mouse, and humans. As would be expected for molecules that regulate gene expression, miRNA levels have been shown to vary between tissues and developmental states, hi addition, one study shows a strong correlation between reduced expression of two miRNAs and chronic lymphocytic leukemia, providing a possible link between miRNAs and cancer (Calin, 2002). Although the field is still young, there is speculation that miRNAs could be as important as transcription factors in regulating gene expression in higher eukaryotes.

There are a few examples of miRNAs that play critical roles in cell differentiation, early development, and cellular processes like apoptosis and fat metabolism, lin-4 and let-7 both regulate passage from one larval state to another during C. elegans development (Ambros, 2003). mir-14 and bantam are drosophila miRNAs that regulate cell death, apparently by regulating the expression of genes involved in apoptosis (Brennecke et ah, 2003, Xu et al, 2003). Mir-14 has also been implicated in fat metabolism (Xu et al, 2003). Lsy-6 and miR-273 are C. elegans miRNAs that regulate asymmetry in cliemosensory neurons (Chang et al, 2004). Another animal miRNA that regulates cell differentiation is miR-181, which guides hematopoietic cell differentiation (Chen et al, 2004). These molecules represent the full range of animal miRNAs with known functions. Enhanced understanding of the functions of miRNAs will undoubtedly reveal regulatory networks that contribute to normal development, differentiation, inter- and intra-cellular communication, cell cycle, angiogenesis, apoptosis, and many other cellular processes. Given their important roles in many biological functions, it is likely that miRNAs will offer important points for therapeutic intervention or diagnostic analysis.

An abundant liver-specific miRNA miR-122 is important for efficient replication of HCV RNA in cultured human hepatoma Huh7 cells stably expressing an HCV replicon (Jopling CL, Yi M, Lancaster AM, Lemon SM, Sarnow P. Science 2005;309:1577-1581). This observation raised much interest in a role of miR-122 in HCV infection and as a potential therapeutic target. As explained herein, current therapy of CHC consists of pegylated INFα (pegIFNα) in combination with ribavirin but the treatment achieves a sustained virological response in only ~55% of patients (Manns MP et al. Lancet 2001;358:958-965; Fried MW et al. N Engl J Med 2002;347:975-982). It was reported recently that levels of miR-122 and several other miRNAs are regulated by IFN in Huh7 hepatoma cells and primary mouse hepatocytes, and that miRNAs might mediate at least some effects of IFN on HCV RNA replication *in vitro* (Pedersen IM et al. Nature 2007;449:919-922). All experiments performed to date assessed a role of miR-122 in HCV replication in cultured cells. To determine the status of miR-122 in patients with chronic hepatitis C (CHC), the present inventors compared levels of miR-122 and other miRNAs in liver biopsies collected from forty-two CHC patients undergoing treatment with pegIFNα and ribavirin. Measurements were performed by quantitative PCR (qPCR), using chemically synthesized miRNAs as standards. The present inventors found that patients who did not respond to therapy with a decrease of the viral load by more than 2 log¹⁰ at week 12, classified as primary non-responders (PNR), had significantly lower levels of miR-122 than patients with a strong response to IFNα and undetectable HCV RNA at week 12 (complete Early Virological Response; cEVR). Differences between PNR and cEVR patients were independent of the liver fibrosis stage, indicating that decrease of miR-122 in PNR patients is not due to a lower proportion of hepatocytes in the biopsy material. Moreover, the difference in miR-122 expression between the groups was still apparent when miR-122 levels where normalized to the hepatocyte specific albumin mRNA, further supporting a direct correlation between miR-122 levels and response to treatment.

Clinical studies have shown that patients with high HCV viral load respond less well to therapy (Manns MP et al. Lancet 2001;358:958-965; Fried MW et al. N Engl J Med 2002;347:975-982). Since miR-122 is important for HCV replication in Huh7 hepatoma cells, the finding of low miR-122 in non-responders by the present inventors was surprising. The present inventors measured HCV RNA in liver and serum of the patients. No correlation was observed between levels of miR-122 and HCV viral load. These results are opposite to the results obtained *in vitro* and do not support a significant role of miR-122 in HCV replication *in vivo* (see for example WO-A-2007/112754, which proposes to inhibit miR-122 for the therapy of HCV). The decreased miR-122 level in non-responders raises a possibility that miR-122 is a negatively regulated IFN target gene. Indeed, expression of miR-122 in biopsies of CHC patients shows negative correlation with expression of known IFN-stimulated genes (ISGs).

In summary, the present surprising results demonstrate that (i) patients non-responding to IFN therapy generally have several fold lower levels of miR-122 than responders; (ii) that administration of pegIFNα has no significant effect on the miR-122 level in patients liver; and (iii) that no correlation exists between intra-hepatic miR-122 and HCV RNA levels. This is unexpected, because based on the HCV replicon studies in Huh7 cells, low levels of miR-122 should decrease viral yields and benefit the therapy. Notwithstanding, the finding that the miR-122 level is significantly lower in PNR than cEVR patients makes miR-122 a convenient marker suitable, for instance together with pre-activated ISGs (Sarasin-Filipowicz M et al. Proc Natl Acad Sci U S A 2008, 105(19):7034-7039; Chen L et al. Gastroenterology 2005;128:1437-1444; Asselah T et al. Gut 2008;57:516-524), for predicting the outcome of IFN therapy in CHC patients. Moreover, the present results also surprisingly demonstrate that (i) patients non-responding to IFN therapy generally have several fold higher levels of miR-296-5p than responders. This demonstrate that miR-296-5p, and more generally miRNAs, can be used for predicting the outcome of IFN therapy in CHC patients.

This led the inventors to realise that a method based on miRNA could be developed to help a clinician decide on a treatment regimen for subjects suffering from a viral infection of the liver. Levels of miRNA, e.g. miR-122 or miR-296-5p, from an infected individual can be compared with gene expression from a control (i.e. a subject without viral infection).

Infected subjects with lower levels of miR-122 and/or higher levels of miR-296-5p (as compared to the control) would be unlikely to benefit from the use of IFN in a treatment regimen (i.e. these individuals would not be expected to have an RVR) whereas infected subjects for whom levels of miR-122 and/or miR-296-5p was mostly unaltered or elevated, compared to control expression, are likely to benefit from IFN therapy and have an RVR. The inventors were surprised to make these correlations because the *in vitro* results known in the art predicted the contrary.

While there have been previous studies of gene expression levels in "responder" and "non-responder" subjects with a viral infection of the liver, e.g. Chen et al (2005) Gastroenterology 128, 1437-1444, the research conducted as part of the present invention studied a very much broader set of genes in order to determine which would act as prognostic markers. In addition, the present inventors also assessed
whether different miRNAs could be used as prognostic markers. Moreover, the inventors also analysed gene expression and miRNAs levels in samples taken before and after antiviral treatment, and used this information to identify prognostic genetic markers, while previous studies only attempted to correlate treatment outcome to gene expression levels present in samples taken *before* treatment. Thus the data set which lead to the identification of the prognostic genetic markers set out below are considered to be much more complete and robust than that in previous studies.

Accordingly in the first aspect of the invention there is provided a method for determining the likelihood that a subject having a viral infection of the liver will be responsive to antiviral therapy that includes stimulation of Interferon (IFN) activity, the method comprising analysing a sample from the subject for the level of expression of a miRNA and, comparing the level of expression of said miRNA in the sample from the subject to the levels of said miRNA in a control sample.

In some embodiments of the invention, the miRNA is miR122 and a lower level of miR-122 in the sample from the subject as compared to the level of miR-122 in the control sample indicate that the subject is not likely to be responsive to said antiviral therapy.

In some other embodiments of the invention, which embodiments can be combined with those described above, the miRNA is miR-296-5p, and a higher level of miR-296-5p in the sample from the subject as compared to the level of miR-296-5p in the control sample indicate that the subject is not likely to be responsive to said antiviral therapy.

In some embodiment of the invention, an unaltered or elevated level of miR-122, and/or an unaltered or lowered level of miR-296-5p, respectively, in the sample from the subject as compared to the level of miR-122, or miR-296-5p, respectively, in the control sample can indicate that the subject is likely to be responsive to said antiviral therapy.

It is to be understood that the methods of the invention are not restricted to the use of one miRNA only but also encompass any combination of the level of expression of any miRNA with those of miR-122 and/or miR-296-5p, or the combination of any relevant miRNA, in particular any miRNA for to have a predictive power for the likelihood that a subject having a viral infection of the liver will be responsive to antiviral therapy

In the methods of the invention, the antiviral therapy can be pegylated IFNα (pegIFNα), optionally combined with ribavirin.

Moreover, in some embodiments of the invention, the viral infection is Hepatitis B virus or Hepatitis C virus infection.

Moreover, liver tissue can be used as sample in the methods of the invention.

Advantageously, the expression of genes, which might be predictive of the subject's response to therapy, can also analysed in the methods of the invention in order to increase the predictive power, for instance some of the genes published by some of the inventors on a previous study (Sarasin-Filipowicz et al., 2008, PNAS, 105(19):7034-7039) Said gene expression can be determined by measuring the amount of mRNA gene transcript in the sample, or the amount of cDNA derived from said mRNA, or by measuring the amount of peptide or polypeptide encoded by the gene in the sample, for instance, using a specific binding molecule.

In some embodimens of the invention, the subject is a mammal, for instance a human being.

Also encompassed is a kit for performing the method of the invention, said kit comprising means for analysing in a sample from a subject the level of expression of a miRNA, e.g. miR-122 or miR-296-5p; and, optionally, means for comparing the level of expression of said miRNA, e.g. miR-122 or miR-296-5p, in the sample from the subject to the levels of said miRNA, e.g. miR-122 or miR-296-5p, in a control sample. The kit can further comprise means for analysing the expression of at least one gene which might be predictive of the subject's response to therapy, eventually comprising one or more specific binding molecules that can target molecules representative of said at least one gene which might be predictive of the subject's response to therapy, wherein said specific binding molecule is an oligonucleotide probe, antibody, or aptamer ; and, optionally, means for comparing expression of the genes in the sample to expression of the same genes in a control sample.

It will be appreciated that the methods of the invention will be of great benefit to clinicians. IFN is a protein growth factor and pharmaceutical preparations containing IFN are expensive to manufacture. It is therefore very important for a clinician to be confident that IFN is being used in an appropriate and cost-effective way. Furthermore, independent of the cost, it is often desirable to eliminate a viral infection of the liver as quickly as possible. It is therefore wasting time (which could be spent utilising alternative therapies) if a clinician administers IFN and subsequently discovers that it has no beneficial effects. The methods of the invention are therefore of great assistance to a clinician because he can identify two populations of subjects. One population will show lower levels of miR-122 and /or higher levels of miR-296-5p as compared to control and will therefore probably not benefit from treatment with IFN. The other population, with normal or elevated levels of miR-122, and/ or normal or lower expression levels of miR-296-5p, as compared to control, will benefit from therapy with IFN.

A method of influencing the response of a subject having a viral infection of the liver to antiviral therapy that includes stimulation of Interferon (IFN) activity, the method comprising modulating the levels of a miRNA, for instance miR-122 or miR-296-5p is described.

The levels of the miRNA, for instance miR-122 or miR-296-5p could be regulated by any suitable method known in the art. Examples of such methods can be found in e.g. WO-A-2006/137941, WO-A-2007/021896 or WO-A-2007/090073.

By "antiviral therapy" we mean any treatment regimen for reducing viral infection that involves the stimulation of IFN activity. Such a regimen may involve the use of compounds that stimulate Type I IFN activity and/or induce IFN stimulated genes (ISGs). The therapy may involve treatment with IFN *per se* or other IFN receptor agonists. For example the therapy may utilise pegylated IFNα (pegIFNα).

The therapy may involve the stimulation of IFN activity alone. However the inventors have found that the method according to the invention is particularly useful for predicting the effectiveness of an antiviral therapy that comprises the use of a combination therapy comprising a stimulator of IFN activity in conjunction with a known antiviral agent. Many antiviral agents are known to the art and the method of the invention can be used to evaluate the effectiveness of a number of different combination therapies. However the inventors have found that the method of the invention has particular value for predicting the effectiveness of therapy with a stimulator of IFN activity used in conjunction with the antiviral agent ribavirin.

In one embodiment, the method of the invention is used to predict the usefulness of pegIFNα and ribavirin as an antiviral therapy.

The method of the invention may be utilised to evaluate the effectiveness of treatments for a number of different viral infections of the liver, including Hepatitis B virus and Hepatitis C virus infections. In one embodiment, the method is utilised to evaluate the effectiveness of therapies for Hepatitis C Virus (HCV) infection. The inventors have found that the method of the invention is particularly useful for distinguishing between subjects that will be expected to have a rapid virological response (RVR) and those which will not (non-RVR).

Samples representative of the levels of miR-122 in a subject that may be used in accordance with the present invention encompass any sample that may provide information as to said levels of miR-122 in the subject.

Examples of suitable samples include biopsies, samples excised during surgical procedures, blood samples, urine samples, sputum samples, cerebrospinal fluid samples, and swabbed samples (such as saliva swab samples). It will be appreciated that the source of the sample will depend upon which type of viral infection the subject may have.

In one embodiment, samples are from liver tissue. Liver samples have been found to be particularly instructive when the method is applied to assessing subjects with HCV infection. The inventors were surprised to find that RVR could be distinguished from non-RVR subjects by analysing levels of miR-122.

Suitable samples may include tissue sections such as histological or frozen sections. Methods by which such sections may be prepared in such a way as to be able to provide information representative of the levels of expression of miRNA, for instance miR-122 or miR-296-5p, in the subject from which the section is derived will be well known to those skilled in the art, and should be selected with reference to the technique that it is intended to use when investigating gene expression.

Although the use of samples comprising a portion of tissue from the subject is contemplated, it may generally be preferred that the sample representative of levels of the miRNA, for instance miR-122 or miR-296-5p, comprise a suitable extract taken from such a tissue, said extract being capable of investigation to provide information regarding said levels of said miRNA, for instance miR-122 or miR-296-5p, in the subject. Suitable protocols which may be used for the production of tissue extracts capable of providing information regarding gene expression in a subject will be well known to those skilled in the art. Preferred protocols may be selected with reference to the manner in which gene expression is to be investigated.

In the case of samples derived from liver suitable preparation steps are disclosed in the Example.

By "control sample" we mean a sample, equivalent to that from the subject, that has been derived from an individual that is not suffering from a viral infection of the liver. Although equivalent tissue or organ samples, constituting control samples, or extracts from such samples, may be used directly as the source of information regarding levels of miRNA, for instance miR-122 or miR-296-5p, in the control sample, it will be appreciated, and generally be preferred, that information regarding the expression of the levels of miRNA, for instance miR-122 or miR-296-5p, in an "ideal" control sample be provided in the form of reference data. Such reference data may be provided in the form of tables indicative of the levels of miRNA, for instance miR-122 or miR-296-5p, in the chosen control tissue. Alternatively, the reference data may be supplied in the form of computer software containing retrievable information indicative of the levels of miRNA, for instance miR-122 or miR-296-5p, in the chosen control tissue. The reference data may, for example, be provided in the form of an algorithm enabling comparison of expression of at least the levels of miRNA, for instance miR-122 or miR-296-5p, in the subject with expression of this miRNA in the control tissue sample.

In the event that the levels of miRNA, for instance miR-122 or miR-296-5p, in a control sample is to be investigated via processing of a tissue or organ sample constituting the control sample, it is beneficial that such processing is conducted using the same methods used to process the sample from the subject. Such parallel processing of subject samples and control samples allows a greater degree of confidence that comparisons of gene expression in these tissues will be normalised relative to one another (since any artefacts associated with the selected method by which tissue is processed and gene expression investigated will be applied to both the subject and control samples).

The method according to the invention may involve the analysis of at least the levels of miR-122. The finding that altered levels of miR-122 may be used in determining the effectiveness of an antiviral therapy is surprising. In view of the results published in the prior art, iit was total unexpected that lower levels of miR-122 would be associated with poor response to subsequent IFN treatment.

In previous studies, the inventors have identified different genes, the expression levels of which can be prognostic markers for the outcome of antiviral therapy. The present invention relating to miR-122 can be combined with said genes in order to provide a yet more robust prediction.

The presence, absence and/or levels of miRNA, for instance miR-122 or miR-296-5p, may be investigated will generally be detected using suitable probe molecules. Such detection will provide information as to the presence, absence and/or levels of miRNA, for instance miR-122 or miR-296-5p, and thereby allow comparison between the levels of miRNA, for instance miR-122 or miR-296-5p, occurring in the subject and those occurring in the control sample. Probes will generally be capable of binding specifically to the miRNA, for instance miR-122 or miR-296-5p, directly or indirectly. Binding of such probes may then be assessed and correlated with the levels of the miRNA, for instance miR-122 or miR-296-5p, to allow an effective prognostic comparison between the subject and the control.

By "altered expression" we include where the gene expression is both elevated or reduced in the sample when compared to the control, as discussed above.

Conversely by "unaltered expression" we include where the gene expression is not elevated or reduced in the sample when compared to the control, as discussed above.

An assessment of the levels of a miRNA, for instance miR-122 or miR-296-5p, and of whether a gene expression is altered or unaltered can be made using routine methods of statistical analysis.

The target molecule may be peptide or polypeptide. The amount of peptide or polypeptide can be determined using a specific binding molecule, for instance an antibody. In a preferred instance, the amount of certain target proteins present in a sample may be assessed with reference to the biological activity of the target protein in the sample. Assessment and comparison of expression in this manner is particularly suitable in the case of protein targets having enzyme activity. Suitable techniques for the measurement of the amount of a protein target present in a sample include, but are not limited to, aptamers and antibody-based techniques, such as radio-immunoassays (RIAs), enzyme-linked immunoassays (ELISAs) and Western blotting.

It will be understood that "nucleic acids" or "nucleic acid molecules" for the purposes of the present invention refer to deoxyribonucleotide or ribonucleotide polymers in either single-or double-stranded form. Furthermore, unless the context requires otherwise, these terms should be taken to encompass known analogues of natural nucleotides that can function in a similar manner to naturally occurring nucleotides.

Furthermore it will be understood that target nucleic acids suitable for use in accordance with the invention need not comprise "full length" nucleic acids (e.g. full length gene transcripts), but need merely comprise a sufficient length to allow specific binding of probe molecules.

In an embodiment of the method of the invention, samples may be treated to isolate RNA target molecules by a process of lysing cells taken from a suitable sample (which may be achieved using a commercially available lysis buffer such as that produced by Qiagen Ltd.) followed by centrifugation of the lysate using a commercially available nucleic acid separation column (such as the RNeasy midi spin column produced by Qiagen Ltd). Other methods for RNA extraction include variations on the phenol and guanidine isothiocyanate method of Chomczynski, P. and Sacchi, N. (1987) Analytical Biochemistry 162, 156. "Single Step Method of RNA Isolation by Acid Guanidinium Thiocyanate-Phenol-Chloroform Extraction." RNA obtained in this manner may constitute a suitable target molecule itself, or may serve as a template for the production of target molecules representative of the levels of miR-122.

In the case of assessing the expression of chosen gene in order to have a more robust prediction value, it may be preferred that RNA derived from a subject or control sample may be used as substrate for cDNA synthesis, for example using the Superscript System (Invitrogen Corp.). The resulting cDNA may then be converted to biotinylated cRNA using the BioArray RNA Transcript labelling Kit (Enzo Life Sciences Inc.) and this cRNA purified from the reaction mixture using an RNeasy mini kit (Qiagen Ltd). mRNA, representative of gene expression, may be measured directly in a tissue derived from a subject or control sample, without the need for mRNA extraction or purification. For example, mRNA present in, and representative of gene expression in, a subject or control sample
of interest may be investigated using appropriately fixed sections or biopsies of such a tissue. The use of samples of this kind may provide benefits in terms of the rapidity with which comparisons of expression can be made, as well as the relatively cheap and simple tissue processing that may be used to produce the sample. *In situ* hybridisation techniques represent preferred methods by which gene expression may be investigated and compared in tissue samples of this kind. Techniques for the processing of tissues of interest that maintain the availability of RNA representative of gene expression in the subject or control sample are well known to those of skill in the art. However, techniques by which mRNAs representative of gene expression in a subject or control sample may be extracted and collected are also well known to those skilled in the art, and the inventors have found that such techniques may be advantageously employed in accordance with the present invention. Samples comprising extracted mRNA from a subject or control sample may be preferred for use in the method of the third aspect of the invention, since such extracts tend to be more readily investigated than is the case for samples comprising the original tissues. For example, suitable target molecules allowing for comparison of gene expression may comprise the total RNA isolated from a sample of tissue from the subject, or a sample of control tissue. Furthermore, extracted RNA may be readily amplified to produce an enlarged mRNA sample capable of yielding increased information on gene expression in the subject or control sample. Suitable examples of techniques for the extraction and amplification of mRNA populations are well known, and are considered in more detail below.

By way of example, methods of isolation and purification of nucleic acids to produce nucleic acid targets suitable for use in accordance with the invention are described in detail in Chapter 3 of Laboratory Techniques in Biochemistry and Molecular Biology: Hybridization With Nucleic Acid Probes, Part I. Theory and Nucleic Acid Preparation, P. Tijssen, ed. Elsevier, N.Y. (1993).

In the event that it is desired to amplify the nucleic acid targets prior to investigation and comparison of gene expression it may be preferred to use a method that maintains or controls for the relative frequencies of the amplified nucleic acids in the subject or control tissue from which the sample is derived.

Suitable methods of "quantitative" amplification are well known to those of skill in the art. One well known example, quantitative PCR, involves simultaneously co-amplifying a control sequence whose quantities are known to be unchanged between control and subject samples. This provides an internal standard that may be used to calibrate the PCR reaction.

In addition to the methods outlined above, the skilled person will appreciate that any technology coupling the amplification of gene-transcript specific product to the generation of a signal may also be suitable for quantitation. A preferred example employs convenient improvements to the polymerase chain reaction (US 4683195 and 4683202) that have rendered it suitable for the exact quantitation of specific mRNA transcripts by incorporating an initial reverse transcription of mRNA to cDNA. Further key improvements enable the measurement of accumulating PCR products in real-time as the reaction progresses.

In many cases it may be preferred to assess the degree of gene expression in subject or control samples using probe molecules capable of indicating the presence of target molecules in the relevant sample.

Probes may be selected with reference to the product (direct or indirect) of gene expression to be investigated. Examples of suitable probes include oligonucleotide probes, antibodies, aptamers, and binding proteins or small molecules having suitable specificity.

Oligonucleotide probes can be used as probes. The generation of suitable oligonucleotide probes is well known to those skilled in the art (Oligonucleotide synthesis: Methods and Applications, Piet Herdewijn (ed) Humana Press (2004)). Oligonucleotide and modified oligonucleotides are commercially available from numerous companies.

For the purposes of the present description, an oligonucleotide probe may be taken to comprise an oligonucleotide capable of hybridising specifically to a nucleic acid target molecule of complementary sequence through one or more types of chemical bond. Such binding may usually occur through complementary base pairing, and usually through hydrogen bond formation. Suitable oligonucleotide probes may include natural (i.e., A, G, C, or T) or modified bases (7-deazaguanosine, inosine, etc.). In addition, a linkage other than a phosphodiester bond may be used to join the bases in the oligonucleotide probe(s), so long as this variation does not interfere with hybridisation of the oligonucleotide probe to its target. Thus, suitable oligonucleotide probes may be peptide nucleic acids in which the constituent bases are joined by peptide bonds rather than phosphodiester linkages.

As explained herein, microRNA molecules ("miRNAs") are generally 21 to 22 nucleotides in length, though lengths of 17 and up to 25 nucleotides have been reported. The miRNAs are each processed from a longer precursor RNA molecule ("precursor miRNA"). Precursor miRNAs are transcribed from non-protein-encoding genes. The precursor miRNAs have two regions of complementarity that enables them to form a stem-loop- or fold-back-like structure, which is cleaved by an enzyme called Dicer in animals. Dicer is ribonuclease III-like nuclease. The processed miRNA is typically a portion of the stem.

The processed miRNA (also referred to as "mature miRNA") become part of a large complex to down-regulate a particular target gene. Examples of animal miRNAs include those that imperfectly basepair with the target, which halts translation (Olsen et al, 1999; Seggerson et al, 2002). SiRNA molecules also are processed by Dicer, but from a long, double-stranded RNA molecule. SiRNAs are not naturally found in animal cells, but they can function in such cells in a RNA-induced silencing complex (RISC) to direct the sequence-specific cleavage of an mRNA target (Denli et al, 2003).

The study of endogenous miRNA molecules is for instance described in U.S. Patent Application 60/575,743. Synthetic miRNAs miRNAs are apparently active in the cell when the mature, single-stranded RNA is bound by a protein complex that regulates the translation of mRNAs that hybridize to the miRNA. Introducing exogenous RNA molecules that affect cells in the same way as endogenously expressed miRNAs requires that a single-stranded RNA molecule of the same sequence as the endogenous mature miRNA be taken up by the protein complex that facilitates translational control. A variety of RNA molecule designs have been evaluated. Three general designs that maximize uptake of the desired single-stranded miRNA by the miRNA pathway have been identified. An RNA molecule with an miRNA sequence having at least one of the three designs is referred to as a synthetic miRNA.

Synthetic miRNAs of the invention comprise, in some embodiments, two RNA molecules wherein one RNA is identical to a naturally occurring, mature miRNA. The RNA molecule that is identical to a mature miRNA is referred to as the active strand. The second RNA molecule, referred to as the complementary strand, is at least partially complementary to the active strand. The active and complementary strands are hybridized to create a double-stranded RNA, called the synthetic miRNA, that is similar to the naturally occurring miRNA precursor that is bound by the protein complex immediately prior to miRNA activation in the cell. Maximizing activity of the synthetic miRNA requires maximizing uptake of the active strand and minimizing uptake of the complementary strand by the miRNA protein complex that regulates gene expression at the level of translation. The molecular designs that provide optimal miRNA activity involve modifications to the complementary strand.

Two designs incorporate chemical modifications in the complementary strand. The first modification involves creating a complementary RNA with a chemical group other than a phosphate or hydroxyl at its 5' terminus. The presence of the 5' modification apparently eliminates uptake of the complementary strand and subsequently favors uptake of the active strand by the miRNA protein complex. The 5' modification can be any of a variety of molecules including NH2, NHCOCH3, biotin, and others.

The second chemical modification strategy that significantly reduces uptake of the complementary strand by the miRNA pathway is incorporating nucleotides with sugar modifications in the first 2-6 nucleotides of the complementary strand. It should be noted that the sugar modifications consistent with the second design strategy can be coupled with 5' terminal modifications consistent with the first design strategy to further enhance synthetic miRNA activities.

A third synthetic miRNA design involves incorporating nucleotides in the 3' end of the complementary strand that are not complementary to the active strand.

Hybrids of the resulting active and complementary RNAs are very stable at the 3' end of the active strand but relatively unstable at the 5' end of the active strand. Studies with siRNAs indicate that 5' hybrid stability is a key indicator of RNA uptake by the protein complex that supports RNA interference, which is at least related to the miRNA pathway in cells. The judicious use of mismatches in the complementary RNA strand significantly enhances the activity of the synthetic miRNA.

As explained herein, the term "miRNA" generally refers to a single-stranded molecule, but in specific embodiments, molecules implemented in the invention will also encompass a region or an additional strand that is partially (between 10 and 50% complementary across length of strand), substantially (greater than 50% but less than 100% complementary across length of strand) or fully complementary to another region of the same single-stranded molecule or to another nucleic acid. Thus, nucleic acids may encompass a molecule that comprises one or more complementary or self- complementary strand(s) or "complement(s)" of a particular sequence comprising a molecule. For example, precursor miRNA may have a self-complementary region, which is up to 100% complementary.

Synthetic miRNAs typically comprise two strands, an active strand that is identical in sequence to the mature miRNA that is being studied and a complemenrtary strand that is at least partially complementary to the active strand. The active strand is the biologically relevant molecule and should be preferentially taken up by the complex in cells that modulates translation either through mRNA degradation or translational control. Preferential uptake of the active strand has two profound results: (1) the observed activity of the synthetic miRNA increases dramatically and (2) non-intended effects induced by uptake and activation of the complementary strand are essentially eliminated. Several synthetic miRNA designs can be used to ensure the preferential uptake of the active strand.

The introduction of a stable moiety other than phosphate or hydroxyl at the 5' end of the complementary strand impairs its activity in the miRNA pathway. This ensures that only the active strand of the synthetic miRNA will be used to regulate translation in the cell. 5' modifications include, but are not limited to, NH2, biotin, an amine group, a lower alkylamine group, an acetyl group, 2 O-Me, DMTO, fluoroscein, a thiol, or acridine or any other group with this type of functionality.

Other sense strand modifications. The introduction of nucleotide modifications like 2'-0Me, NH2, biotin, an amine group, a lower alkylamine group, an acetyl group, DMTO, fluoroscein, a thiol, or acridine or any other group with this type of functionality in the complementary strand of the synthetic miRNA can eliminate the activity of the complementary strand and enhance uptake of the active strand of the miRNA.

As with siRNAs (Schwarz 2003), the relative stability of the 5' and 3' ends of the active strand of the synthetic miRNA apparently determines the uptake and activation of the active by the miRNA pathway. Destabilizing the 5' end of the active strand of the synthetic miRNA by the strategic placement of base mismatches in the 3' end of the complementary strand of the synthetic miRNA enhances the activity of the active strand and essentially eliminates the activity of the complementary strand.

miR-122, also called miRNA-1222, a 22-nucleotide microRNA, is derived from a liver-specific noncoding polyadenylated RNA transcribed from the gene *hcr.* The exact sequence of miR-122 as well as the adjacent secondary structure within the hcr mRNA are conserved from mammalian species back to fish. Levels of miR-122 in the mouse liver increase to half maximal values around day 17 of embryogenesis, and reach near maximal levels of 50,000 copies per average cell before birth (it makes up 70% of all miRNA) (Chang et al., 2004; Lagos-Quintana et al., 2002). It is one of many tissue-specific microRNAs thought to be important for establishing patterns of gene expression that may be responsible for maintaining the differentiated state of a tissue (Lagos-Quintana et al., 2002; Lim et al., 2005).

The phrase "hybridising specifically to" as used herein refers to the binding, duplexing, or hybridising of an oligonucleotide probe preferentially to a particular target nucleotide sequence under stringent conditions when that sequence is present in a complex mixture (such as total cellular DNA or RNA). In one embodiment, a probe may bind, duplex or hybridise only to the particular target molecule.

The term "stringent conditions" refers to conditions under which a probe will hybridise to its target subsequence, but minimally to other sequences. In some embodiments, a probe may hybridise to no sequences other than its target under stringent conditions. Stringent conditions are sequence-dependent and will be different in different circumstances. Longer sequences hybridise specifically at higher temperatures.

In general, stringent conditions may be selected to be about 5°C lower than the thermal melting point (Tm) for the specific sequence at a defined ionic strength and pH. The Tm is the temperature (under defined ionic strength, pH, and nucleic acid concentration) at which 50% of the oligonucleotide probes complementary to a target nucleic acid hybridise to the target nucleic acid at equilibrium. As the target nucleic acids will generally be present in excess, at Tm, 50% of the probes are occupied at equilibrium. By way of example, stringent conditions will be those in which the salt concentration is at least about 0.01 to 1.0 M Na⁺ ion concentration (or other salts) at pH 7.0 to 8.3 and the temperature is at least about 30° C for short probes (e.g., 10 to 50 nucleotides). Stringent conditions may also be achieved with the addition of destabilizing agents such as formamide.

Oligonucleotide probes may be used to detect complementary nucleic acid sequences (i.e., nucleic acid targets) in a suitable representative sample. Such complementary binding forms the basis of most techniques in which oligonucleotides may be used to detect, and thereby allow comparison of, expression of particular genes. Some suitable technologies permit the parallel quantitation of the expression of multiple genes and include technologies where amplification and quantitation of species are coupled in real-time, such as the quantitative reverse transcription PCR technologies and technologies where quantitation of amplified species occurs subsequent to amplification, such as array technologies.

Array technologies involve the hybridisation of samples, representative of the subject or control sample, with a plurality of oligonucleotide probes wherein each probe preferentially hybridises to a disclosed gene or genes, or miRNA. Array technologies provide for the unique identification of specific oligonucleotide sequences, for example by their physical position (e.g., a grid in a two-dimensional array as commercially provided by Affymetrix Inc.) or by association with another feature (e.g. labelled beads as commercially provided by Illumina Inc or Luminex Inc). Oligonuleotide arrays may be synthesised *in situ* (e.g. by light directed synthesis as commercially provided by Affymetrix Inc) or pre-formed and spotted by contact or ink-jet technology (as commercially provided by Agilent or Applied Biosystems). It will be apparent to those skilled in the art that whole or partial cDNA sequences may also serve as probes for array technology (as commercially provided by Clontech).

Oligonucleotide probes may be used in blotting techniques, such as Southern blotting or northern blotting, to detect and compare gene expression (for example by means of cDNA or mRNA target molecules representative of gene expression). Techniques and reagents suitable for use in Southern or northern blotting techniques will be well known to those of skill in the art. Briefly, samples comprising DNA (in the case of Southern blotting) or RNA (in the case of northern blotting) target molecules are separated according to their ability to penetrate a gel of a material such as acrylamide or agarose. Penetration of the gel may be driven by capillary action or by the activity of an electrical field. Once separation of the target molecules has been achieved these molecules are transferred to a thin membrane (typically nylon or nitrocellulose) before being immobilized on the membrane (for example by baking or by ultraviolet radiation). Gene expression may then be detected and compared by hybridisation of oligonucleotide probes to the target molecules bound to the membrane.

In certain circumstances the use of traditional hybridisation protocols for comparing gene expression may prove problematic. For example blotting techniques may have difficulty distinguishing between two or more gene products or miRNAs of approximately the same molecular weight since such similarly
sized products are difficult to separate using gels. Accordingly, in such circumstances it may be preferred to compare gene expression using alternative techniques, such as those described below.

Gene expression in a sample representing gene expression and/or levels of miRNAs in a subject may be assessed with reference to global transcript levels within suitable nucleic acid samples by means of high-density oligonucleotide array technology. Such technologies make use of arrays in which oligonucleotide probes are tethered, for example by covalent attachment, to a solid support. These arrays of oligonucleotide probes immobilized on solid supports represent preferred components to be used in the methods and kits of the invention for the comparison of gene expression. Large numbers of such probes may be attached in this manner to provide arrays suitable for the comparison of expression of large numbers of genes or miRNAs. Accordingly it will be recognised that such oligonucleotide arrays may be particularly preferred in embodiments where it is desired to compare expression of more than one miRNA and/or gene.

Other suitable methodologies that may be used in the comparison of nucleic acid targets representative of gene expression include, but are not limited to, nucleic acid sequence based amplification (NASBA); or rolling circle DNA amplification (RCA).

It is usually desirable to label probes in order that they may be easily detected. Examples of detectable moieties that may be used in the labelling of probes or targets suitable for use in accordance with the invention include any composition detectable by spectroscopic, photochemical, biochemical, immunochemical, electrical, optical or chemical means. Suitable detectable moieties include various enzymes, prosthetic groups, fluorescent materials, luminescent materials, bioluminescent materials, radioactive materials and colourimetric materials. These detectable moieties are suitable for incorporation in all types of probes or targets that may be used in the methods of the invention unless indicated to the contrary.

Examples of suitable enzymes include horseradish peroxidase, alkaline phosphatase, beta-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride, phycoerythrin, texas red, rhodamine, green fluorescent protein, and the like; an example of a luminescent material includes luminol; examples of bioluminescent materials include luciferase, luciferin, and aequorin; examples of suitable radioactive material include ¹²⁵I, ¹³¹I, ³⁵S, ³H, ¹⁴C, or ³²P; examples of suitable colorimetric materials include colloidal gold or coloured glass or plastic (e.g., polystyrene, polypropylene, latex, etc.) beads.

Means of detecting such labels are well known to the skilled person. For example, radiolabels may be detected using photographic film or scintillation counters; fluorescent markers may be detected using a photodetector to detect emitted light. Enzymatic labels are typically detected by providing the enzyme with a substrate and detecting the reaction product produced by the action of the enzyme on the substrate, and colorimetric labels are detected by simply visualizing the coloured label.

In one embodiment of the invention fluorescently labelled probes or targets may be scanned and fluorescence detected using a laser confocal scanner.

In the case of labelled nucleic acid probes or targets suitable labelling may take place before, during, or after hybridisation. In an embodiment, nucleic acid probes or targets are labelled before hybridisation. Fluorescence labels are also suitable and, where used, quantification of the hybridisation of the nucleic acid probes to their nucleic acid targets is by quantification of fluorescence from the hybridised fluorescently labelled nucleic acid. Quantitation may be from a fluorescently labelled reagent that binds a hapten incorporated into the nucleic acid.

In an embodiment of the invention analysis of hybridisation may be achieved using suitable analysis software, such as the Microarray Analysis Suite (Affymetrix Inc.).

Effective quantification may be achieved using a fluorescence microscope which can be equipped with an automated stage to permit automatic scanning of the array, and which can be equipped with a data acquisition system for the automated measurement, recording and subsequent processing of the fluorescence intensity information. Suitable arrangements for such automation are conventional and well known to those skilled in the art.

In an embodiment, the hybridised nucleic acids are detected by detecting one or more detectable moieties attached to the nucleic acids. The detectable moieties may be incorporated by any of a number of means well known to those of skill in the art. However, in an embodiment, such moieties are simultaneously incorporated during an amplification step in the preparation of the sample nucleic acids (probes or targets). Thus, for example, polymerase chain reaction (PCR) using primers or nucleotides labelled with a detectable moiety will provide an amplification product labelled with said moiety. In an alternative embodiment, transcription amplification using a fluorescently labelled nucleotide (e.g. fluorescein-labelled UTP and/or CTP) incorporates the label into the transcribed nucleic acids.

Alternatively, a suitable detectable moiety may be added directly to the original nucleic acid sample (e.g., mRNA, polyA mRNA, cDNA, etc. from the tissue of interest) or to an amplification product after amplification of the original nucleic acid is completed. Means of attaching labels such as fluorescent labels to nucleic acids are well known to those skilled in the art and include, for example nick translation or end-labelling (e.g. with a labeled RNA) by kinasing of the nucleic acid and subsequent attachment (ligation) of a nucleic acid linker joining the sample nucleic acid to a label (such as a suitable fluorophore).

Although the method of the invention is most suitable for use in association with human subjects it will be appreciated that it may also be useful in determining a course of treatment of viral infection in non-human animals (e.g. horses, dogs, cattle, camels).

In previous studies, the inventors established that the endogenous IFN system is constantly activated in many infected patients. Moreover, the inventors were surprised to correlate patients with a pre-activated IFN system with a poor response to IFN therapy. This finding is counter-intuitive, because one would expect that an active innate immune system would help to eliminate the virus during IFNα therapy.

The inventors analysed ISG expression in liver biopsies and further concluded that there are patients where HCV surprisingly induces (does not block) the endogenous IFN system, and there are patients where HCV does not induce (may be by cleaving TRIF and/or Cardif) the endogenous IFN system, but that this difference has no impact on the ability of HCV to maintain a chronic infection.

In patients without a pre-activation of the IFN system, the inventors found that pegIFNa2b induced within 4 hours a robust (sub-) maximal up-regulation of many ISGs in the liver. Surprisingly, such high ISG expression levels were already present in the pretreatment biopsies of patients that later did not show a rapid virological response at week 4.

It was also found that the pre-activation of the endogenous IFN system was found more frequently in liver biopsies of patients infected with HCV genotype 1 (and 4) than with genotype 2 or 3. This is intriguing because it is well known that genotype 2 and 3 infections can be cured in over 80% of the patients, compared to less than 50% of the patients with genotype 1. The inventors finding that the frequency and degree of pre-activation of the endogenous IFN system depends on the HCV genotype provides an explanation for the different treatment susceptibility of HCV genotypes.

The inventors realised that these data establish that HCV interferes with IFN signalling and thereby impairs the response to therapy. Moreover, an inhibition of IFNα signalling by HCV explains why the strong pre-activation of the endogenous IFN system does not lead to a spontaneous elimination of HCV. The inventors do not wish to be bound by any hypothesis but believe this means that IFNα would not induce an antiviral state in the hepatocytes that are infected with HCV. The up-regulation of ISGs observed in the liver of many patients would then occur only in the non-infected hepatocytes. The strong induction of ISGs found in liver biopsies is compatible with such a model, because there are more non-infected that infected hepatocytes. IFNβ production would occur in the hepatocytes infected with a virus that is not successful in cleaving Cardif and/or TRIF. Because of the HCV induced inhibition of the Jak-STAT pathway, the secreted IFNβ would not induce an antiviral state in these infected hepatocytes, but only in non-infected neighbor cells.

By "reduces" we mean that agent is effective for reducing the stimulation of ISGs such that the expression levels of ISGs are not significantly different to expression levels in control tissues.

The agents may be used in the treatment of a number of different viral infections of the liver, including Hepatitis B virus and Hepatitis C virus infections. In one embodiment, the agents are used to prevent or reduce Hepatitis C Virus (HCV) infection.

Examples of agents which may be used include where the agent may bind to the IFNα polypeptide and prevent IFN functional activity, e.g. antibodies and fragments and derivatives thereof (e.g. domain antibodies or Fabs). Alternatively the agent may act as a competitive inhibitor to IFN system by acting as an antagonist at IFNα receptors (e.g. IFNAR1, IFNAR2a, b, or c). Alternatively the agent may inhibit enzymes or other molecules in the IFN pathway. Alternatively the agent may bind to mRNA encoding IFNα polypeptide in such a manner as to lead to a reduction in that mRNA and hence a reduction in IFNα polypeptide. Alternatively the agent may bind to a nucleic sequence encoding IFNα in such a manner that it leads to a reduction in the amount of transcribed mRNA encoding IFNα polypeptide. For instance the agent may bind to coding or non-coding regions of the IFNα gene or to DNA 5' or 3' of the IFN and thereby reduce expression of the protein.

There are a number of different human Interferon α polypeptide sequences. A consensus sequence has been determined. This information can be used by the skilled person to develop a binding agent to IFNα polypeptide.

When the agents binds to IFNα polypeptide, it is preferred that the agent binds to an epitope defined by the protein that has been correctly folded into its native form. It will be appreciated, that there can be some sequence variability between species and also between genotypes. Accordingly other preferred epitopes will comprise equivalent regions from variants of the gene. Equivalent regions from further IFN polypeptides can be identified using sequence similarity and identity tools, and database searching methods, outlined above.

Antibodies may be produced as polyclonal sera by injecting antigen into animals. For instance, polyclonal antibodies may be raised by inoculating an animal (e.g. a rabbit) with antigen (e.g. all or a fragment of the IFNα polypeptide) using techniques known to the art.

Alternatively the antibody may be monoclonal. Conventional hybridoma techniques may be used to raise such antibodies. The antigen used to generate monoclonal antibodies may be the same as would be used to generate polyclonal sera.

In their simplest form, antibodies or immunoglobulin proteins are Y-shaped molecules usually exemplified by the γ-immunoglobulin (IgG) class of antibodies. The molecule consists of four polypeptide chains two identical heavy (H) chains and two identical (L) chains of approximately 50kD and 25kD each respectively. Each light chain is bound to a heavy chain (H-L) by disulphide and noncovalent bonds. Two identical H-L chain combinations are linked to each other by similar noncovalent and disulphide bonds between the two H chains to form the basic four chain immunoglobulin structure (H-L)₂.

Light chain immunoglobulins are made up of one V-domain (V_{L}) and one constant domain (C_{L}) whereas heavy chains consist of one V-domain and, depending on H chain isotype, three or four C-domains (C_{H}1, C_{H}2, C_{H}3 and C_{H}4).

At the N-terminal region of each light or heavy chain is a variable (V) domain that varies greatly in sequence, and is responsible for specific binding to antigen. Antibody specificity for antigen is actually determined by amino acid sequences within the V-regions known as hypervariable loops or Complementarity Determining Regions (CDRs). Each H and L chain V regions possess 3 such CDRs, and it is the combination of all 6 that forms the antibody's antigen binding site. The remaining V-region amino acids which exhibit less variation and which support the hypervariable loops are called frameworks regions (FRs).

The regions beyond the variable domains (C-domains) are relatively constant in sequence. It will be appreciated that the characterising feature of antibodies according to the invention is the V_{H} and V_{L} domains. It will be further appreciated that the precise nature of the C_{H} and C_{L} domains is not, on the whole, critical. In fact preferred antibodies may have very different C_{H} and C_{L} domains. Furthermore, as discussed more fully below, preferred antibody functional derivatives may comprise the Variable domains without a C-domain (e.g. scFV antibodies).

Antibodies, and particularly mAbs, generated in one species are known to have several serious drawbacks when used to treat a different species. For instance when murine antibodies are used in humans they tend to have a short circulating half-life in serum and may be recognised as foreign proteins by the immune system of a patient being treated. This may lead to the development of an unwanted human anti-mouse antibody (HAMA) response. This is particularly troublesome when frequent administration of an antibody is required as it can enhance its clearance, block its therapeutic effect, and induce hypersensitivity reactions. These factors limit the use of mouse monoclonal antibodies in human therapy and have prompted the development of antibody engineering technology to generate humanised antibodies.

Therefore, where the antibody capable of reducing IFN activity is to be used as a therapeutic agent for treating HCV infections in a human subject, then it is preferred that antibodies and fragments thereof of non-human source are humanised.

Humanisation may be achieved by splicing V region sequences (e.g. from a monoclonal antibody generated in a non-human hybridoma) with C region (and ideally FRs from V region) sequences from human antibodies. The resulting 'engineered' antibodies are less immunogenic in humans than the non-human antibodies from which they were derived and so are better suited for clinical use.

Humanised antibodies may be chimaeric monoclonal antibodies, in which, using recombinant DNA technology, rodent immunoglobulin constant regions are replaced by the constant regions of human antibodies. The chimaeric H chain and L chain genes may then be cloned into expression vectors containing suitable regulatory elements and induced into mammalian cells in order to produce fully glycosylated antibodies. By choosing an appropriate human H chain C region gene for this process, the biological activity of the antibody may be pre-determined. Such chimaeric molecules may be used to treat or prevent cancer.

Further humanisation of antibodies may involve CDR-grafting or reshaping of antibodies. Such antibodies are produced by transplanting the heavy and light chain CDRs of a non-human antibody (which form the antibody's antigen binding site) into the corresponding framework regions of a human antibody.

Humanised antibody fragments represent preferred agents. Human FAbs recognising an epitope on IFNα polypeptide or an IFN receptor may be identified through screening a phage library of variable chain human antibodies. Techniques known to the art (e.g as developed by Morphosys or Cambridge Antibody Technology) may be employed to generate Fabs that may be used as agents. In brief a human combinatorial Fab antibody library may be generated by transferring the heavy and light chain variable regions from a single-chain Fv library into a Fab display vector. This library may yield 2.1 x 10¹⁰ different antibody fragments. The peptide may then be used as "bait" to identify antibody fragments from then library that have the desired binding properties.

Domain antibodies (dAbs) represent another preferred agent that may be used. dAbs are the smallest functional binding unit of antibodies and correspond to the variable regions of either the heavy or light chains of human antibodies. Such dAbs may have a molecule weight of around 13kDa (corresponding to about 1/10 (or less) the size of a full antibody).

Aptamers represent another preferred agent of the third or fourth aspect. Aptamers are nucleic acid molecules that assume a specific, sequence-dependent shape and bind to specific target ligands based on a lock-and-key fit between the aptamer and ligand. Typically, aptamers may comprise either single- or double-stranded DNA molecules (ssDNA or dsDNA) or single-stranded RNA molecules (ssRNA). Aptamers may be used to bind both nucleic acid and non-nucleic acid targets. Accordingly aptamers may be generated that recognise and so reduce the activity or expression of IFNα. Suitable aptamers may be selected from random sequence pools, from which specific aptamers may be identified which bind to the selected target molecules with high affinity. Methods for the production and selection of aptamers having desired specificity are well known to those skilled in the art, and include the SELEX (systematic evolution of ligands by exponential enrichment) process. Briefly, large libraries of oligonucleotides are produced, allowing the isolation of large amounts of functional nucleic acids by an iterative process of *in vitro* selection and subsequent amplification through polymerase chain reaction.

Antisense molecules represent another preferred agent for use to treat patients. Antisense molecules are typically single-stranded nucleic acids, which can specifically bind to a complementary nucleic acid sequence produced by a gene and inactivate it, effectively turning that gene "off". The molecule is termed "antisense" as it is complementary to the gene's mRNA, which is called the "sense" sequence, as appreciated by the skilled person. Antisense molecules are typically are 15 to 35 bases in length of DNA, RNA or a chemical analogue. Antisense nucleic acids have been used experimentally to bind to mRNA and prevent the expression of specific genes. This has lead to the development of "antisense therapies" as drugs for the treatment of cancer, diabetes and inflammatory diseases. Antisense drugs have recently been approved by the US FDA for human therapeutic use. Accordingly, by designing an antisense molecule to polynucleotide sequence encoding IFN polypeptide it would be possible to reduce the expression of IFNα polypeptide in a cell and thereby reduce in IFNα activity and reduce the preactiviation seen in HCV infection. A polynucleotide sequence encoding an IFNα polypeptide is provided in Figure 8.

Small interfering RNA (siRNA), sometimes known as short interfering RNA or silencing RNA, represent further preferred agents for use according to the third or fourth aspects. As set out above, the inventors realised that preactivation of the IFN system is associated with a resistance to antiviral therapy. It is therefore apparent that siRNA molecules that can reduce IFNα expression have great utility in the preparation of medicaments for the treatment of HCV infection. siRNA are a class of 20-25 nucleotide-long RNA molecules are involved in the RNA interference pathway (RNAi), by which the siRNA can lead to a reduction in expression of a specific gene, or specifically interfere with the translation of such mRNA thereby inhibiting expression of protein encoded by the mRNA. siRNAs have a well defined structure: a short (usually 21-nt) double-strand of RNA (dsRNA) with 2-nt 3' overhangs on either end. Each strand has a 5' phosphate group and a 3' hydroxyl (-OH) group. *In vivo* this structure is the result of processing by Dicer, an enzyme that converts either long dsRNAs or hairpin RNAs into siRNAs. siRNAs can also be exogenously (artificially) introduced into cells by various transfection methods to bring about the specific knockdown of a gene of interest. Essentially any gene of which the sequence is known can thus be targeted based on sequence complementarity with an appropriately tailored siRNA. Given the ability to knockdown essentially any gene of interest, RNAi via siRNAs has generated a great deal of interest in both basic and applied biology. There is an increasing number of large-scale RNAi screens that are designed to identify the important genes in various biological pathways. As disease processes also depend on the activity of multiple genes, it is expected that in some situations turning off the activity of a gene with a siRNA could produce a therapeutic benefit. Hence their discovery has led to a surge in interest in harnessing RNAi for biomedical research and drug development. Recent phase I results of therapeutic RNAi trials demonstrate that siRNAs are well tolerated and have suitable pharmacokinetic properties. siRNAs and related RNAi induction methods therefore stand to become an important new class of drugs in the foreseeable future. siRNA molecules designed to nucleic acid encoding IFNα polypeptide can be used to reduce the expression of IFNα and therefore result in a reduction in the preactivation of the IFN system. Hence an embodiment of this aspect is wherein the agent is a siRNA molecule having complementary sequence to IFNα polynucleotide.

Using such information it is straightforward and well within the capability of the skilled person to design siRNA molecules having complementary sequence to IFNα polynucleotide. For example, a simple internet search yields many websites that can be used to design siRNA molecules.

By "siRNA molecule" we include a double stranded 20 to 25 nucleotide-long RNA molecule, as well as each of the two single RNA strands that make up a siRNA molecule.

For instance, the siRNA is used in the form of hairpin RNA (shRNA). Such shRNA may comprise two complementary siRNA molecules that are linked by a spacer sequence (e.g. of about 9 nueclotides). The complementary siRNA molecules may fold such that they bind together.

A ribozyme capable of cleaving RNA or DNA encoding IFNα polypeptide represent another preferred agent of the third or fourth aspect.

A "subject" may be a vertebrate, mammal, domestic animal or human being. It is preferred that the subject to be treated is human. When this is the case the agents may be designed such that they are most suited for human therapy (e.g. humanisation of antibodies as discussed above). However it will also be appreciated that the agents may also be used to treat other animals of veterinary interest (e.g. horses, camels, cattle, dogs or cats).

A "pharmaceutically acceptable vehicle" as referred to herein is any physiological vehicle known to those skilled in the art as useful in formulating pharmaceutical compositions.

The various elements required for a technician to perform the method of the first aspect of the invention may be incorporated in to a kit.

Thus, according to an aspect a kit for determining the likelihood that a subject having a viral infection of the liver will be responsive to antiviral therapy that includes stimulation of Interferon (IFN) activity, comprising:
(i) means for analysing in a sample from a subject the levels of a miRNA, for instance miR-122 or miR-296-5p; and, optionally,
(ii) means for comparing the levels of the miRNA, for instance miR-122 or miR-296-5p, in the sample to the levels of said miRNA, for instance miR-122 or miR-296-5p, in a control sample.

By "means for analysing in a sample from a subject the levels of a miRNA, for instance miR-122 or miR-296-5p" we include the specific binding molecules that can target molecules representative of the levels of the miRNA, for instance miR-122 or miR-296-5p, in the sample. In some embodiments, the specific binding molecule is an oligonucleotide probe, antibody, aptamers, or binding proteins or small molecules mentioned above.

By "means for comparing the levels of the miRNA, for instance miR-122 or miR-296-5p ,in the sample to the levels of said miRNA, for instance miR-122 or miR-296-5p, in a control sample" we include the control samples mentioned above in the first aspect of the invention. We also include the control reference data mentioned therein.

The kit may also comprise:
(iii) relevant buffers and reagents for analysing the level of expression of the miRNA, for instance miR-122 or miR-296-5p.

The buffers and regents provided with the kit may be in liquid form and in some embodiments, provided as pre-measured aliquots. Alternatively, the buffers and regents may be in concentrated (or even powder form) for dilution.

All of the features described herein (including any accompanying claims, abstract and drawings), and/or all of the steps of any method or process so disclosed, may be combined with any of the above aspects in any combination, except combinations where at least some of such features and/or steps are mutually exclusive.

The invention will now be further described with reference to the following Example and figures in which:

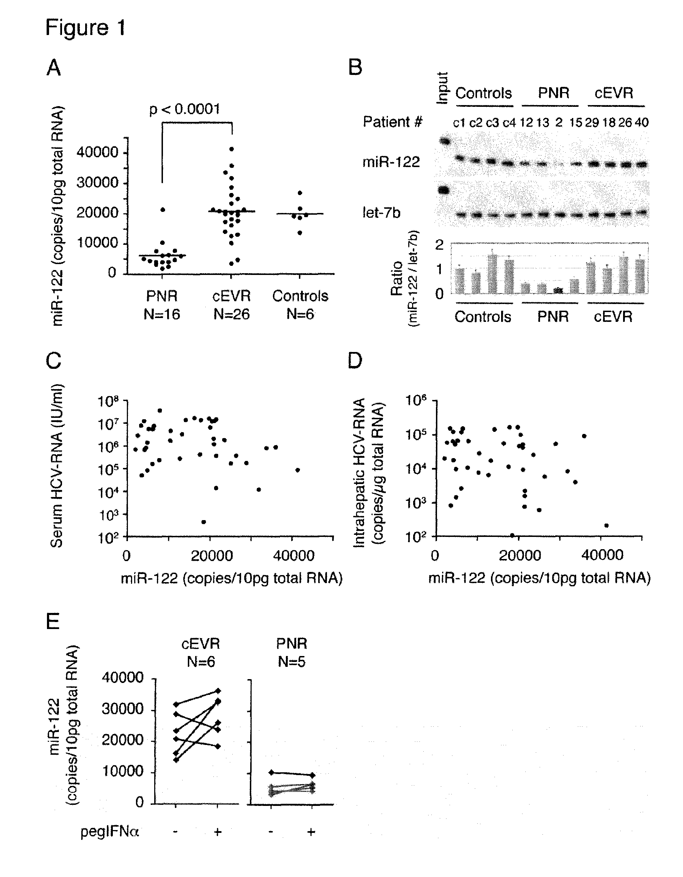

- **Figure 1.**: **miR-122 expression in livers of patients with chronic hepatitis C (CHC). (A) miR-122 levels are significantly lower in primary non-responders (PNR) to pegIFNα therapy than in patients with a complete early virological response (cEVR)**. The amount of miR-122 in the livers of 42 CHC patients and 6 control patients without liver disease was measured by RT-qPCR and is depicted as copies/10pg total RNA. The difference between the 16 PNR and 26 cEVR patients is statistically significant (p<0.0001) as determined by two-tailed Mann Whitney t-test. There is no significant difference between cEVR patients and controls. **(B) RNase protection assay confirms the difference in miR-122 levels between PNR and cEVR patients**. RNA originating from four control patients and eight CHC patients (four PNR and four cEVR; patient numbers, shown at the top of the panels) was analyzed with probes specific for miR-122 (upper panel) and let-7b (middle panel). The bars in the lower panel represent relative ratios between miR-122 and let-7b RNAs (averages from two experiments similar to those shown in upper and lower panels), determined by quantitative Phosporlmaging for each patient. PNR patients show 3-4 fold lower miR-122 levels than cEVR and control patients. **(C) Serum HCV RNA levels do not correlate with intra-hepatic miR-122 expression.** Serum viral load (international units per ml) is plotted on a logarithmic scale (y-axis). The correlation is not significant (p = 0.35), with a correlation coefficient r = 0.15. **(D) Intra-hepatic HCV RNA levels do not correlate with miR-122 expression**. Intra- hepatic HCV RNA (copies per µg total liver RNA) is plotted on a logarithmic scale (y- axis). The correlation is not significant (p = 0.06, correlation coefficient r = -0.29). (E) Administration of PegIFNα does not have a significant effect on miR-122 level in human liver. MiR-122 levels (copies/10pg total RNA) in paired human liver biopsies from six cEVR and five PNR patients with CHC were measured by RT-qPCR. The first liver biopsy was performed before treatment initiation (- pegIFNa). The second biopsy (+ pegIFNα) was performed 4 hours after the first pegIFNα injection. The diagram shows the level of miR-122 in paired liver samples (connected by a line) of each patient.

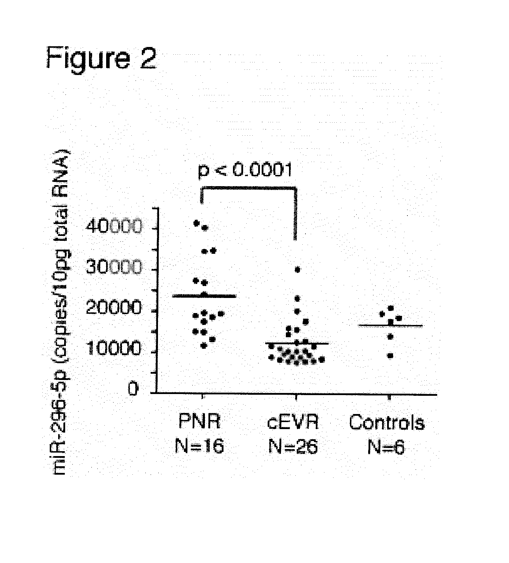

- **Figure 2.**: **miR-296-5p expression in livers of patients with chronic hepatitis C (CHC). miR-296-5p levels are significantly higher in primary non-responders (PNR) to pegIFNα therapy than in patients with a complete early virological response (cEVR).** The amount of miR-296-5p in the livers of 42 CHC patients and 6 control patients without liver disease was measured by RT-qPCR and is depicted as copies/10pg total RNA. The difference between the 16 PNR and 26 cEVR patients is statistically significant (p<0.0001) as determined by two-tailed Mann Whitney t-test.

### Examples

### Patient samples and hepatitis C treatment

From October 2006 to March 2008, patients with CHC referred to the outpatient liver clinic of the University Hospital Basel were asked for permission to use part of their diagnostic liver biopsy for research purposes. 42 patients that were treated with pegylated-IFNα2b (pegIFNα; Peglntron, Essex Chemie AG, Switzerland) and ribavirin (Rebetol, Essex Chemie AG, Switzerland; weight based dosing: <65 kg, 800 mg/d; 65-85 kg, 1 g/d; >85 kg, 1.2 g/d) had sufficient liver biopsy material available for repeated RNA extractions. All of them were Caucasians. 11 of those 42 patients had also agreed to undergo a second liver biopsy 4 hours after the first injection of pegIFNα. The protocol was approved by the Ethics Committee of the University Hospital Basel and written informed consent was obtained from all patients. Serum HCV RNA was quantified before treatment initiation and at weeks 4 and 12 of the treatment using the COBAS AmpliPrep/COBAS Taqman® HCV-Test from Roche Molecular Systems. Histological Grading and Staging of the HCV liver biopsies according to the Metavir classification system was performed at the Pathology Institute of the University Hospital Basel. Non-CHC healthy control tissue was obtained from 6 patients who underwent ultrasound-guided liver biopsies of focal lesions and gave informed consent for a biopsy from the normal liver tissue outside the focal lesion. These patients had normal liver values and the absence of liver pathology was histologically confirmed for all control samples.

### Selection of miRNAs for quantification in liver tissues and Huh7 cells

In addition to miR-122, the miRNAs selected for quantification and analysis of IFN effects in Huh-7 cells and human and mouse liver samples were miR-1, miR-196, miR-296, miR-351, and miR-448, all reported as INF-inducible by Pedersen et al (Pedersen IM et al. Nature 2007;449:919-922). Transfection of each of them, with the exception of miR-1 was also reported to lower the amount of HCV replicon RNA in Huh7 cells (Pedersen IM et al. Nature 2007;449:919-922). Another miRNA reported as leading to the decrease in the HCV replicon RNA yield is miR-431, but this miRNA was not analyzed by us since an assay for its quantification is not available from Applied Biosystems and to date this miRNA has not been identified as expressed in liver or hepatoma cells. Likewise, miR-30 was not analyzed since it exists in multiple forms (miR-30a, b, c, d, and e) but the form analyzed was not specified by Pedersen et al. In addition, overexpression of this miRNAs had no effect on HCV RNA yield (Pedersen IM et al. Nature 2007;449:919-922). Although specific miR-196 and miR-296 forms analyzed by Pedersen et al. have not been indicated, we selected for quantification miR-196b (the two miR-196-specific clones identified in human hepatoma cells represent miR-196b and not miR-196a) and miR-296-5p (the Applied Biosystems Taqman® microRNA Assay, also used by Pedersen et al., is available for miR-296-5p but not for miR-296-3p). Apart from miR-196b, no clones representing other analyzed miRNAs (miR-1, miR-296, miR-351, and miR-448) have been isolated from human liver or hepatoma cells (ref. 3).

Let-7b and miR-15a were selected as control miRNAs levels of which were not expected to be affected in CHC or by the IFN treatment.

### RNA extraction, reverse transcription and Real-Time qPCR quantification of small RNAs

Total and small RNA fractions from Huh7 and Huh7.5 cells were extracted using Trizol and mirVana™ miRNA Isolation Kit (Ambion, Austin, TX), respectively and according to manufacturer's protocol. Total RNA from human or mouse liver was isolated by homogenizing 10-30 mg of frozen tissues in Trizol reagent. RNA concentration was quantified using NanoDrop Spectrophotometer (NanoDrop Technologies, USA).

For analysis of let-7b, miR-15a, miR-122, miR-1, miR-196b, miR-296-5p, miR-351, and miR-448 expression in human and mouse material (sequences of these miRNAs in mouse and human are identical), the Applied Biosystem Taqman® microRNA Assay System (Applied Biosystems, Foster City, CA) was used. Reverse transcriptase (RT) reactions, in final volume of 7.5 µl, contained 1 x RT buffer, 5 ng of RNA, 50 nM miRNA-specific RT primer, 0.25 mM each dNTP, 0.25 U/µl RNase inhibitor, and 3.33 U/µl MultiScribe RT, were incubated for 30 min at 16°C, 30 min at 42°C, 5 min at 85°C and then held at 4°C. All RT reactions, including no-template controls, were run in triplicate. Real-time PCR was performed using an Applied Biosystems Prism 7000 Sequence Detection System. The 10 µl PCR included 0.67 µl RT product, 1× Taqman Universal PCR master mix, and 1 µl of primers and a probe mix of the Taqman® MicroRNA Assay. The reactions were incubated in a 96-well optical plate at 95°C for 5 min, followed by 40 cycles of 95°C for 15 sec and 60°C for 60 sec. The Ct values were determined using default threshold settings. The threshold cycle (Ct) is defined as the fractional cycle number at which the fluorescence passes the fixed threshold.

### Normalization and miRNA copy number estimation.

The Ct values determined for each sample were normalized to the average Ct obtained for U6 RNA, calculated from triplicate reactions. To determine miRNA copy numbers, standard curves were generated, using the polyacrylamide-gel-electrophoresis (PAGE)- purified oligoribonucleotides (Microsynth, Switzerland) corresponding to let-7b, miR-15a, miR-122, miR-1, miR-196b, miR-296-5p, miR-351, and miR-448 miRNAs. Synthetic RNA input ranged from 1.3 aM (equivalent to 7 copies per reaction) to 13 pM (7 × 10⁷ copies per reaction) and the reactions were analyzed by the Taqman® MicroRNA Assay in duplicate, using the Applied Biosystems Prism 7000 as described above. To further validate the standardization performed with isolated synthetic miRNAs, reactions containing 5 ng of total RNA from either HeLa cells or mouse retina were spiked with increasing concentrations (1.3 aM to 13 pM) of synthetic miR-122 and analyzed by the Taqman® MicroRNA Assay. MiR-122 is not expressed in HeLa and mouse retinal cells as determined by control reactions without addition of synthetic miR-122 RNA. No differences were observed between standard curves obtained in the absence or presence of the "carrier" RNA (data not shown).

The Ct values obtained from different reactions were converted to absolute copy number of specific miRNAs per 10 pg of total RNA from liver tissue or per single Huh7 cell, using appropriate standardization curves. When indicated, recovery of miRNAs was further normalized for levels of the hepatocyte-specific albumin mRNA. The primers used for the human albumin RT-qPCR were TTGGAAAAATCCCACTGCAT (SEQ ID NO:1) and CTCCAAGCTGCTCAAAAAGC (SEQ ID NO:2). The SYBR-PCR reactions were performed using the SYBR green PCR master mix (Applied Biosystems, Foster City, CA)

To determine copy numbers of individual miRNAs per Huh7 cell, recovery of total RNA per cell was calculated by three different procedures. For the first two procedures, cells were counted using the CASY1 Cell Counter according to manufacturer's protocol (Scharfe System, Germany) and RNA from a known number of cells was extracted using either Trizol or mirVana kit. In the third procedure, cells were counted under the microscope and RNA extracted by Trizol. All three procedures were performed in triplicates using different numbers of cells for RNA extraction. Samples were spiked with a ³²P-labelled RNA to determine RNA recovery. The there procedures yielded following amounts of total RNA per cell: 13.8 +/- 3.5 pg, 14.52 +/- 2.65 pg, and 13,98 +/- 2.37 pg. An average value of 14.1 pg/cell was used for further calculations. This value is lower from 25 pg of RNA per cell used previously by Chang et al. for calculation of the miR-122 content in hepatocytes and hepatoma cells. However, it falls in the range of values calculated for mouse T lympocytes at different developmental stages (0.67 to 6.82 pg/cell) or mouse ES cells (20 pg/cell).

To determine small RNA (< 200 nt) content per cell, total Huh7 cell RNA obtained by different procedures was subjected to fractionation by mirVana™ kit. It was calculated that a single Huh7 cell contains 1.125 pg of small RNA (average of determination ranging from 1.05 to1.2 pg per cells). This value, which is similar to that determined by Brown et al. was used to recalculate numbers of the miRNA copies per 10 pg of small RNA (as determined in Brown et al., also using the Applied Biosystem Taqman® Assays) to miRNA copies per cell. We note that the values of miRNA copies per cell determined by the Applied Biosystem Taqman® Assays tend to be lower then those determined by RNase protection (our unpublished results). This is due to the fact that the Applied Biosystem Taqman® System does not detect individual 3'-end sequence variants of a specific miRNA with identical efficacy (unpublished results).

### miRNA detection by RNase Protection Assay

RNase Protection Assays (RPA) were performed using the mirVana miRNA probe construction and detection kits (Ambion), according to the manufacturer's instructions. Primers used for preparation of the RPA probes were: let-7b, TGAGGTAGTAGGTTGTGTGGTTCCTGTCTC (SEQ ID NO:3); and miR-122, TGGAGTGTGACAATGGTGTTTGTCCTGTCTC (SEQ ID NO:4). Briefly, RPA reactions contained 5 fmol (100,000 cpm) of internally-labeled and PAGE-purified RPA probe and 1 µg of total liver RNA. Hybridization was carried out overnight at 42°C and RNase A/T1 digestion was at 37°C for 20 min. The protected fragments were analyzed by 12% PAGE under denaturing conditions, followed by Phosphorlmager (Typhoon, Molecular Dynamics) quantification.

### Determination of ISG mRNA levels

Total RNA extracted from human or mouse liver tissue was used for quantification of "classical" ISG mRNAs (PKR, OAS1, STAT1 and SOCS1) as a measure of IFN action. The RNA was reverse transcribed by Moloney murine leukemia virus reverse transcriptase (Promega Biosciences, Inc., Wallisellen, Switzerland) in the presence of random hexamers (Promega) and deoxynucleoside triphosphates. Reactions were incubated for 5 min at 70°C, then for 1 h at 37°C, and were stopped by heating at 95°C for 5 min. The SYBR-PCR reactions were performed using the SYBR green PCR master mix (Applied Biosystems) and primers spanning the exon-intron junctions to avoid amplification of genomic DNA. The following primers were used for mouse (m) genes: ribosomal protein L-19 (mRPL-19), ATCCGCAAGCCTGTGACTGT (SEQ ID NO:5) and TCGGGCCAGGGTGTTTTT (SEQ ID NO:6); mPKR, TGATAACGAAAACAAGGTGGATTG (SEQ ID NO:7) and ACAAGGCCTATGTAGTTACCAACGA (SEQ ID NO:8); mOAS1, CACCCA GTGAGGGTCTCCAA (SEQ ID NO:9) and CCCTTGAGTGTGGTGCCTTT (SEQ ID NO:10); mSTAT1, CGGCGCAGAGAGATTTGC (SEQ ID NO:11) and AGCTGAAACGACTGGC TCTCA (SEQ ID NO:12). The primers for human (h) genes were: glyceraldehyde-3-phosphate dehydrogenase (hGAPDH); GCTCCTCCTGTTCGACAGTCA (SEQ ID NO:13) and ACCTTCCCCATGGTGTCTGA (SEQ ID NO:14), hPKR, TGATTATCTGCGTGCATTTTGG (SEQ ID NO:15) and GCTGAAAGATCACCAGCCATTT (SEQ ID NO:16); hOAS1, TGATGCCCTGGGTCAGTTG (SEQ ID NO:17) and TCGGTGCACTCCTCGATGA (SEQ ID NO:18); hSOCS1, CCCCTTCTGTAGGATGGTAGCA (SEQ ID NO:19) and TGCTGTGGAGACTGCATTGTC (SEQ ID NO:20); hSTAT1 TCCCCAGGCCCTTGTTG (SEQ ID NO:21) and CAAGCTGCTGAAGTTGGTACCA (SEQ ID NO:22); hIP10, CGATTCTGATTTGCTGCCTTAT (SEQ ID NO:23) and GCAGGTACAGCGTACGGTTCT (SEQ ID NO:24); hUSP18, CTCAGTCCCGACGTGGAACT (SEQ ID NO:25) and ATCTCTCAAGCGCCATGCA (SEQ ID NO:26); hIFI27, CCTCGGGCAGCCTTGTG (SEQ ID NO:27) and AATCCGGAGAGTCCAGTTGCT (SEQ ID NO:28). The difference in the cycle threshold (ΔCt) value was derived by subtracting the Ct value for hGAPDH or mRPL-19, serving as internal controls, from the Ct value for transcripts of interest. All reactions were run in duplicate, using an Applied Biosystems Prism 7000 Sequence Detection System. mRNA expression levels were calculated relative to hGAPDH or mRPL-19 from the ΔCt values, using the formula 2^{-ΔCt}. The change of expression in paired liver biopsy samples was calculated as a fold change according to the formula 2^(ΔCt B-1-ΔCt B-2).

### Quantification of intra-hepatic HCV RNA

1 µg of total RNA isolated from biopsies of 42 CHC and 6 control patients was reverse transcribed according to manufacturer's instructions using a pathogen specific RT primer mix (Quantification Kit for HCV, PrimerDesign Ltd, Southampton, UK) designed for the *in vitro* quantification of all HCV subtypes. HCV RNA was quantified according to manufacturer's instructions using a pathogen specific primer/probe mix (PrimerDesign Ltd) and the Taqman® Universal PCR Master Mix (Applied Biosystems, manufactured by Roche, New Jersey, USA). Fluorescence was detected through the FAM channel of the Applied Biosystems 7000 Sequence Detection System and copies of HCV RNA per 1 µg of total RNA were calculated according to the standard curve obtained using the pathogen positive control template (PrimerDesign Ltd).

## Claims

1. A method for determining the likelihood that a subject having a viral infection of the liver will be responsive to antiviral therapy comprising Interferon (IFN) treatment, the method comprising:
(a) analyzing a tissue sample from the subject having the viral infection for the level of expression of miR-122 and/or miR-296-5p and,
(b) comparing the level of expression of miR-122 and/or miR-296-5p in the tissue sample from the subject having the viral infection to the level of expression of miR-122 and/or miR-296-5p in a control tissue sample from a subject without viral infection,
wherein a significantly lower level of miR-122 in the sample from the subject having the viral infection as compared to the level of miR-122 in the control sample from an individual that is not suffering from a viral infection of the liver indicates that the subject having the viral infection is not likely to respond to said antiviral therapy; and
wherein a significantly higher level of miR-296-5p in the sample from the subject having the viral infection as compared to the level of miR-296-5p in the control sample from the subject without viral infection indicate that the subject having the viral infection is not likely to respond to said antiviral therapy.

2. The method of any of the previous claims wherein the antiviral therapy includes pegylated INFα (pegINFα) combined with ribavirin.

3. The method of any of the previous claims wherein the viral infection is Hepatitis B virus or Hepatitis C virus infection.

4. The method of claim 4 wherein the virus is Hepatitis C virus.

5. The method of any of the previous claims wherein the tissue sample from the subject having the viral infection comprises liver tissue.

6. The method of any of the previous claims wherein the subject is human.

## Revendications

1. Procédé de détermination de la probabilité qu'un sujet présentant une infection virale du foie réponde à une thérapie antivirale comprenant un traitement à l'interféron (IFN), le procédé comprenant les étapes consistant à :
(a) analyser un échantillon de tissu du sujet présentant l'infection virale pour connaître le niveau d'expression de miR-122 et/ou de miR-296-5p, et
(b) comparer le niveau d'expression de miR-122 et/ou de miR-296-5p dans l'échantillon de tissu du sujet présentant l'infection virale avec le niveau d'expression de miR-122 et/ou de miR-296-5p dans un échantillon de tissu témoin d'un sujet sans infection virale,
où un niveau significativement moindre de miR-122 dans l'échantillon du sujet présentant l'infection virale par rapport au niveau de miR-122 dans l'échantillon témoin d'un individu qui ne souffre pas d'une infection virale du foie indique que le sujet présentant l'infection virale n'est pas susceptible de répondre à ladite thérapie antivirale ; et
où un niveau significativement supérieur de miR-296-5p dans l'échantillon du sujet présentant l'infection virale par rapport au niveau de miR-296-5p dans l'échantillon témoin d'un individu qui ne souffre pas de l'infection virale du foie indique que le sujet présentant l'infection virale n'est pas susceptible de répondre à ladite thérapie antivirale.

2. Procédé selon l'une quelconque des revendications précédentes, dans lequel la thérapie antivirale comprend de l'INFα PEGylé combiné à de la ribavirine.

3. Procédé selon l'une quelconque des revendications précédentes, dans lequel l'infection virale est une infection par le virus de l'hépatite B ou par le virus de l'hépatite C.

4. Procédé selon la revendication 3, dans lequel le virus est le virus de l'hépatite C.

5. Procédé selon l'une quelconque des revendications précédentes, dans lequel l'échantillon de tissu du sujet présentant l'infection virale comprend un tissu hépatique.

6. Procédé selon l'une quelconque des revendications précédentes, dans lequel le sujet est un humain.

## Patentansprüche

1. Verfahren zur Bestimmung der Wahrscheinlichkeit, dass ein eine virale Infektion der Leber aufweisendes Individuum auf eine antivirale Therapie, die eine Behandlung mit Interferon (IFN) umfasst, anspricht, wobei das Verfahren folgendes umfasst:
a) Analysieren einer Gewebeprobe von dem die virale Infektion aufweisenden Individuum hinsichtlich des Niveaus der Expression von miR-122 und/oder miR-296-5p und
b) Vergleichen des Expressionsniveaus von miR-122 und/oder miR-296-5p in der Gewebeprobe von dem die virale Infektion aufweisenden Individuum mit dem Expressionsniveau von miR-122 und/oder miR-296-5p in einer Kontrollgewebeprobe von einem Individuum ohne virale Infektion,
wobei ein signifikant niedrigeres Niveau von miR-122 in der Probe von dem die virale Infektion aufweisenden Individuum im Vergleich zu dem Niveau von miR-122 in der Kontrollprobe von einem Individuum, das nicht an einer viralen Infektion der Leber leidet, anzeigt, dass das die virale Infektion aufweisende Individuum wahrscheinlich nicht auf die genannte antivirale Therapie ansprechen wird,
wobei ein signifikant höheres Niveau von miR-296-5p in der Probe von dem die virale Infektion aufweisenden Individuum im Vergleich zu dem Niveau von miR-296-5p in der Kontrollprobe von dem Individuum ohne virale Infektion anzeigt, dass das die virale Infektion aufweisende Individuum wahrscheinlich nicht auf die genannte antivirale Therapie ansprechen wird.

2. Verfahren nach einem der vorhergehenden Ansprüche, wobei die antivirale Therapie pegyliertes INFα (pegINFα) in Kombination mit Ribavirin umfasst.

3. Verfahren nach einem der vorhergehenden Ansprüche, wobei die virale Infektion eine Infektion mit Hepatitis B-Virus oder Hepatitis C-Virus ist.

4. Verfahren nach Anspruch 3, wobei das Virus Hepatitis C-Virus ist.

5. Verfahren nach einem der vorhergehenden Ansprüche, wobei die Gewebprobe von dem die virale infektion aufweisenden Individuum Lebergewebe umfasst.

6. Verfahren nach einem der vorhergehenden Ansprüche, wobei das Individuum ein Mensch ist.


### SEQUENCE LISTING

<110> Novartis Forschungsstiftung, Zweigniederlassung Friedrich Miescher Institute for Biomdeical Research University Hospital, Basel
<120> Prediction of Antiviral Therapy Response
<130> 52675
<160> 28
<170> PatentIn version 3.3
<210> 1
   <211> 20
   <212> DNA
   <213> Homo sapiens
<400> 1
   ttggaaaaat cccactgcat 20
<210> 2
   <211> 20
   <212> DNA
   <213> Homo sapiens
<400> 2
   ctccaagctg ctcaaaaagc 20
<210> 3
   <211> 30
   <212> DNA
   <213> Homo sapiens
<400> 3
   tgaggtagta ggttgtgtgg ttcctgtctc 30
<210> 4
   <211> 31
   <212> DNA
   <213> Homo sapiens
<400> 4
   tggagtgtga caatggtgtt tgtcctgtct c 31
<210> 5
   <211> 20
   <212> DNA
   <213> Mus musculus
<400> 5
   atccgcaagc ctgtgactgt 20
<210> 6
   <211> 18
   <212> DNA
   <213> Mus musculus
<400> 6
   tcgggccagg gtgttttt 18
<210> 7
   <211> 24
   <212> DNA
   <213> Mus musculus
<400> 7
   tgataacgaa aacaaggtgg attg 24
<210> 8
   <211> 25
   <212> DNA
   <213> Mus musculus
<400> 8
   acaaggccta tgtagttacc aacga 25
<210> 9
   <211> 20
   <212> DNA
   <213> Mus musculus
<400> 9
   cacccagtga gggtctccaa 20
<210> 10
   <211> 20
   <212> DNA
   <213> Mus musculus
<400> 10
   cccttgagtg tggtgccttt 20
<210> 11
   <211> 18
   <212> DNA
   <213> Mus musculus
<400> 11
   cggcgcagag agatttgc 18
<210> 12
   <211> 21
   <212> DNA
   <213> Mus musculus
<400> 12
   agctgaaacg actggctctc a 21
<210> 13
   <211> 21
   <212> DNA
   <213> Homo sapiens
<400> 13
   gctcctcctg ttcgacagtc a 21
<210> 14
   <211> 20
   <212> DNA
   <213> Homo sapiens
<400> 14
   accttcccca tggtgtctga 20
<210> 15
   <211> 22
   <212> DNA
   <213> Homo sapiens
<400> 15
   tgattatctg cgtgcatttt gg 22
<210> 16
   <211> 22
   <212> DNA
   <213> Homo sapiens
<400> 16
   gctgaaagat caccagccat tt 22
<210> 17
   <211> 19
   <212> DNA
   <213> Homo sapiens
<400> 17
   tgatgccctg ggtcagttg 19
<210> 18
   <211> 19
   <212> DNA
   <213> Homo sapiens
<400> 18
   tcggtgcact cctcgatga 19
   <210> 19
   <211> 22
   <212> DNA
   <213> Homo sapiens
<400> 19
   gctgaaagat caccagccat tt 22
<210> 20
   <211> 21
   <212> DNA
   <213> Homo sapiens
<400> 20
   tgctgtggag actgcattgt c 21
<210> 21
   <211> 17
   <212> DNA
   <213> Homo sapiens
<400> 21
   tccccaggcc cttgttg 17
<210> 22
   <211> 22
   <212> DNA
   <213> Homo sapiens
<400> 22
   caagctgctg aagttggtac ca 22
<210> 23
   <211> 22
   <212> DNA
   <213> Homo sapiens
<400> 23
   cgattctgat ttgctgcctt at 22
<210> 24
   <211> 21
   <212> DNA
   <213> Homo sapiens
<400> 24
   gcaggtacag cgtacggttc t 21
<210> 25
   <211> 20
   <212> DNA
   <213> Homo sapiens
<400> 25
   ctcagtcccg acgtggaact 20
<210> 26
   <211> 19
   <212> DNA
   <213> Homo sapiens
<400> 26
   atctctcaag cgccatgca 19
<210> 27
   <211> 17
   <212> DNA
   <213> Homo sapiens
<400> 27
   cctcgggcag ccttgtg 17
<210> 28
   <211> 21
   <212> DNA
   <213> Homo sapiens
<400> 28
   aatccggaga gtccagttgc t 21